# EUROPEAN PATENT APPLICATION

(11) **EP 1 566 177 A1**
(43) Date of publication of application: **24.08.2005**
(21) Application number: 04020050.3
(22) Date of filing: 24.08.2004
(51) Int. Cl.: A61K 31/663, A61P 17/02, A61K 7/48

(54) **The use of bisphosphonates for treating skin by stimulating of collagen synthesis**

(30) Priority: 23.02.2004 KR 2004012012
(71) Applicant: Tigen Biotech Co., Ltd., Gyeonggi-do 451-884 (KR)
(72) Inventor: Kim, Jin, Seodaemun-gu Seoul 120-101 (KR); Yook, Jong In, Yongsan-gu Seoul 140-885 (KR); Kim, Nam Hee, Mapo-gu Seoul 121-870 (KR); Ryu, Ju Kyoung, Jongro-gu Seoul 110-030 (KR)
(74) Representative: GROSSE BOCKHORNI SCHUMACHER

(57) **Abstract**

The present invention relates to a novel use of bisphosphonate, and more particularly to a composition for increasing collagen synthesis comprising bisphosphonate as an active ingredient, as well as the use thereof. The inventive composition comprising the bisphosphonate promotes collagen synthesis, and thus, when it is applied to the skin, it reduces wrinkles of the skin and makes the skin elastic. For this reason, the inventive composition will be useful as cosmetics for the prevention or improvement of skin aging or a wound-healing agent. Furthermore, the inventive composition can remarkably increase the collagen synthesis of fibroblasts *in vitro*, and thus, can be effectively used in the production of products containing collagen.

### SEQUENCE LISTING

<110> Tigen Biotech Co.,Ltd
<120> The Use of Bisphosphonates for Stimulating of Collagen Synthesis
<160> 2
<170> KopatentIn 1.71
<210> 1
   <211> 17
   <212> DNA
   <213> Artificial Sequence
<220>
   <223> sense primer
<400> 1
   gacgagacca agaactg 17
<210> 2
   <211> 20
   <212> DNA
   <213> Artificial Sequence
<220>
   <223> antisense primer
<400> 2
   ccatccaaac cactgaaacc 20

## Description

### TECHNICAL FIELD

The present invention relates to a novel use of bisphosphonate. More particularly, the present invention relates to a composition for increasing collagen synthesis, which comprises bisphosphonate as an active ingredient, as well as cosmetics for the prevention or improvement of skin aging, comprising the composition, and a wound-healing agent comprising the composition. In addition, the present invention relates to a method for increasing collagen synthesis *in vitro* using bisphosphonate.

### BACKGROUND ART

Collagen is a major matrix protein which is synthesized in fibroblasts and present in extracellular matrix. Collagen is an important protein representing 25% of the total weight of proteins in the human body and has a triple helical structure. Collagen is the major fibrous constituent of skin, tendon, bone, cartilage, blood vessel and the like. The known major functions of collagen are to maintain mechanical strength of the skin, resistance of connective tissue, binding strength of tissue, and cell adhesion, and to induce cell cleavage and differentiation (upon the growth of organisms or wound healing) (Van der Rest *et. al*., 1990).

Collagen, a major protein constituting the skin, particularly in tandem with elastin, is reduced by aging and photoaging due to UV exposure. Due to this reduction in the collagen protein, the skin loses elasticity and becomes wrinkled (Arthur K. Balin *et. al., Aging and the skin,* 1989). Particularly, it is reported that the amount of type 1 collagen is reduced due to the photoaging of the skin, and wrinkles of the skin exposed to sunlight have a close connection with the amount of collagen (Chen S *et al., J. Inv. Der.,* 98; 248-254, 1992). As a result of recent extensive studies on skin aging, the important functions of collagen in the skin have been established. Also, it was verified that when collagen metabolism is activated by an increase in collagen synthesis by skin fibroblasts, dermal matrix components would be increased, thus reducing wrinkles, increasing skin elasticity, strengthening the skin and healing wounds.

As living standards increase, an interest in beautiful skin has also been increased. This interest in skin beauty is being concentrated mainly on the fields of whitening, brown spot removal, wrinkle removal, etc. Particularly a variety of injection compositions for wrinkle removal are actively developed. Most of such injection compositions contain collagen as a main component. However, since the direct substituted with halogen; (d) a cycloalkylamino with 5-7 carbon atoms; and (e) a saturated 5- or 6-membered nitrogen-containing heterocyclyl with 1-2 heteroatoms.

In the definition of the substituent X, the alkyl group of each of the alkylamino and dialkylamino substituents may have 1-6 carbon atoms and may be independently coupled to a dialkylamino group. The term "heterocyclyl" means a saturated or unsaturated, 5- to 7-membered heterocyclyl group having 1 or 2 rings and 1-3 heteroatoms independently selected from N, O and S.

Unless otherwise described or stated, the term "aryl" means a substituted or unsubstituted phenyl, furyl, thienyl or pyridyl group, or an optional fused ring system (e.g., naphthyl) of such groups. The term "substituted aryl" means the aryl group as defined above substituted with one or more of alkyl, alkoxy, halogen, amino, thiol, nitro, hydroxy, acyl, aryl and cyano groups.

Examples of bisphosphonate, i.e., bisphosphonic acid and a pharmaceutically acceptable salt thereof or a hydrate thereof, which can be used as an active ingredient in the present invention, include the following compounds (a) to (s):
(a) 4-amino-1-hydroxybutylidene-1,1-bisphospnonic acid (alendronic acid) or alendronate;
(b) N-methyl-4-amino-1-hydroxybutylidene-1,1-bisphosphonic acid;
(c) 4-(N,N-dimethylamino)-1-hydroxybutylidene-1,1-bisphosphonic acid;
(d) 3-amino-1-hydroxypropylidene-1,1-bisphosphonic acid (pamidronic acid) or pamidronate;
(e) 3-(N-methyl-N-pentyl)-amino-1-hydroxypropane-1,1-bisphosphonic acid (ivandronic acid);
(f) [3-(N-methyl-N-pentyl)-amino-1 -hydroxypropane-1,1 -bisphosphonic acid, monosodium salt, monohydrate] (ivandronate);
(g) 1-hydroxy-3-(N-methyl-N-pentylamino)-propylidene-1,1-bisphosphonic acid;
(h) 1-hydroxy-2-[3-pyridinyl]-ethylidene-1,1-bisphosphonic acid (licedronic acid) or licedronate;
(i) 4-(hydroxymethylene-1,1-bisphosphonic acid)-piperidine;
(j) cycloheptylaminomethylene-1,1-bisphosphonic acid (cimadronic acid) or cimadronate;
(k) 1,1-dichloromethylene-1,1-bisphosphonic acid(clodronic acid) or disodium salt(clodronate);
(l) 1-hydroxy-3-(1-pyrrolidinyl)-propylidene-1,1-bisphosphonic acid (EB-1053);
(m) 1-hydroxyethylidene-1,1-bisphosphonic acid (etidronic acid) or etidronate;
(n) 6-amino-1-hydroxyhexylidene-1,1-bisphosphonic acid (neridronic acid) or neridronate;
(o) 3-(dimethylamino)-1-hydroxypropylidene-1,1-bisphosphonic acid (olpadronic acid) or olpadronate;
(p) [2-(2-pyridinyl)-ethylidene]-1,1-bisphosphonic acid (piridronic acid) or piridronate;
(q) 1-(4-chlorophenylthio)methylidene-1,1-bisphosphonic acid (tiludronic acid) or tiludronate;
(r) 1 -hydroxy-2-(1H-imidazol-1-yl)-ethylidene-1,1-bisphosphonic acid (zoledronic acid) or zoledronate; and
(s) [1-hydroxy-2-imidazo-(1,2-a)pyridin-3-ylethylidene]-bisphosphonic acid; as well as pharmaceutically acceptable salts thereof or hydrates thereof.

Preferred examples of bisphosphonate used in the present invention are aminobisphosphonates, for example, ivandronic acid, clodronic acid, etidronic acid, licedronic acid, zoledronic acid, tiludronic acid, pamidronic acid, cimadronic acid and alendronic acid, as well as pharmaceutically acceptable salts thereof or hydrates thereof. Most preferred examples are pamidronic acid, alendronic acid, pharmaceutically acceptable salts thereof and hydrates thereof.

All the bisphosphonates of the present invention and preparing methods thereof are well known in literature and commercially easily available. The bisphosphonic acid or a pharmaceutically acceptable salt thereof, which is used as a pharmaceutically active ingredient, is described in US Patent No. 4,666,895, US Patent No. 4,719,203, EP-A No. 252,504, EP-A No. 252,505, US Patent No. 4,777,163, US Patent No. 5,002,937 and US Patent No. 4,971,958.

The preparing methods of bisphosphonate can be found in US Patent No. 3,962,432, US Patent No. 4,054,598, US Patent No. 4,267,108, US Patent No. 4,327,039, US Patent No. 4,407,761, US Patent No. 4,621,077, US Patent No. 4,624,947, US Patent No. 4,746,654, US Patent No. 4,922,077, US Patent No. 4,970,335, US Patent No. 5,019,651, US Patent No. 4,761,406, US Patent No. 4,876,248, EP-A No. 252,504 and J. Org. Chem., 32, 4111, 1967.

Examples of a pharmaceutically acceptable salts of the bisphosphonate used in the present invention include ammonium salts; alkali or alkaline earth metal salts, for example, potassium salts, sodium salts, calcium salts and magnesium salts; salts with organic base, such as dicyclohexylamine salts; and salts with amino acids, such as arginine or lysine. Physiologically acceptable non-toxic salts are preferred. Such salts may be prepared by any method known in the art, as described in EP-A No. 252,504 or US Patent No. 4,922,077.

The bisphosphonate of the present invention can be used in the form of a geometrical isomer, such as a cis-trans isomer, an optical isomer, or any suitable isomer mixture.

The bisphosphonate of the present invention may also be used in the form of any suitable hydrate and may contain a solvent which is used for crystallization.

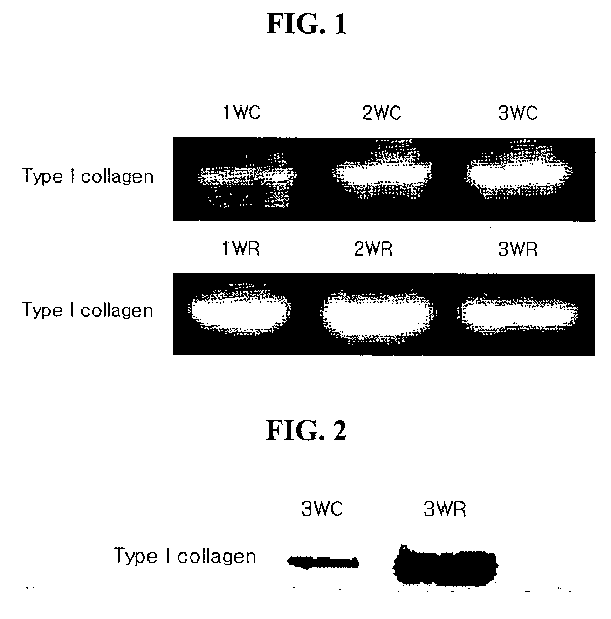

In one embodiment of the present invention, fibroblasts were isolated from human oral epithelium, and cultured in a medium containing pamidronate and vitamin C which is known to induce collagen formation. The culture results showed that the intracellular levels of the mRNA and expression of type 1 collagen known to function to maintain the mechanical strength, elasticity and structure of the skin were increased (see FIGS. 1 and 2).

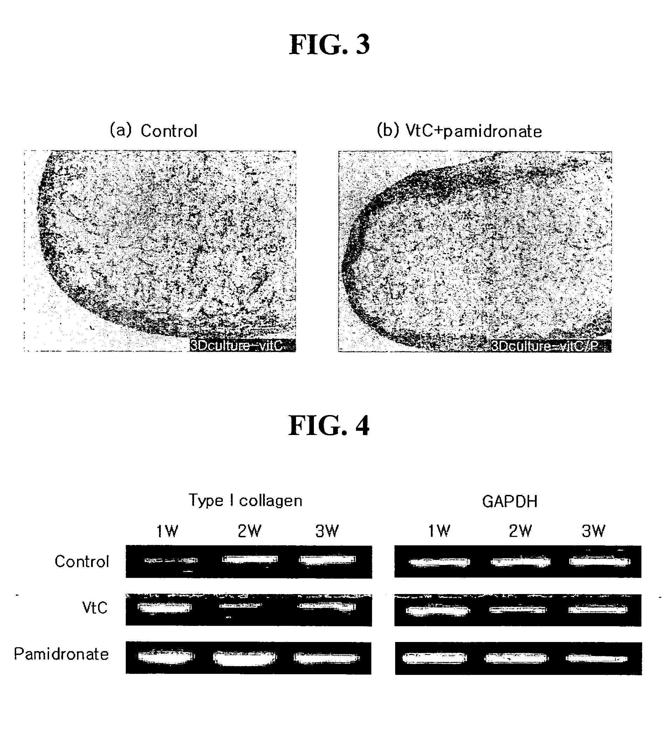

In another embodiment of the present invention, fibroblasts were isolated from human dermis, and the isolated fibroblasts were three-dimensionally cultured, namely placed in a three-dimensional structure and then cultured. The culture results showed that, when the fibroblasts were cultured in a medium containing pamidronate and vitamin C, collagen synthesis was significantly increased as compared with when they were cultured in a medium containing vitamin C alone (see FIG. 3).

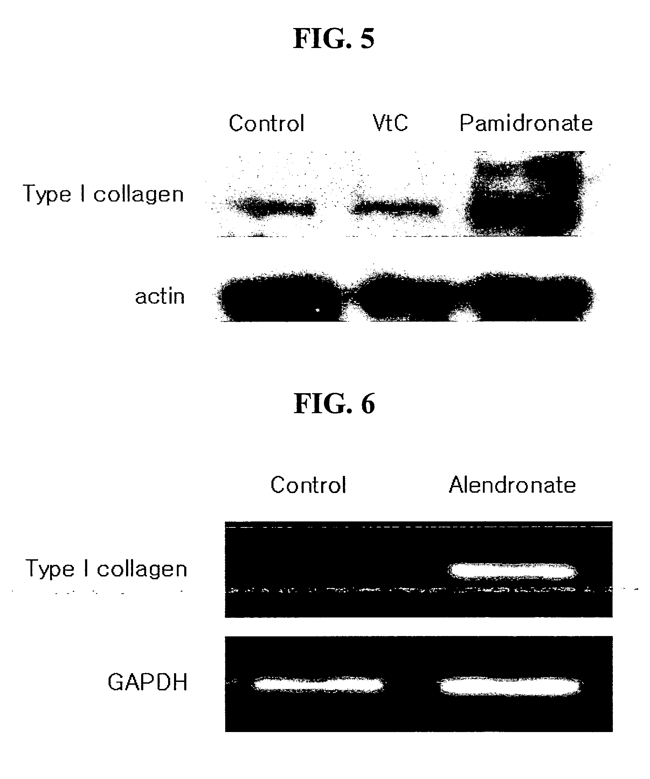

In another embodiment of the present invention, in order to examine whether the single addition of bisphosphonate also has the effect of increasing collagen synthesis in fibroblasts, fibroblasts were isolated from human dermis and cultured in a medium containing pamidronate alone without vitamin C. The culture results showed that even in the case of single addition of pamidronate, the synthesis of type 1 collagen was increased, and also remarkably increased as compared with the case of single addition of vitamin C (see FIG. 5).

Furthermore, the addition of both vitamin C and pamidronate increased the synthesis of type 1 collagen in fibroblasts, but showed a difference in the mRNA level of type 1 collagen according to an increase in culture time. Namely, in the case of addition of vitamin C, the mRNA level of type 1 collagen was rapidly increased at the initial stage of culture, but then reduced with an increase in culture time (see FIG. 4). On the other hand, in the case of addition of pamidronate, the mRNA level of type 1 collagen was high regardless of culture time (see FIG. 4).

Accordingly, it was confirmed that pamidronate remarkably increases the mRNA level and synthesis of type 1 collagen in fibroblasts.

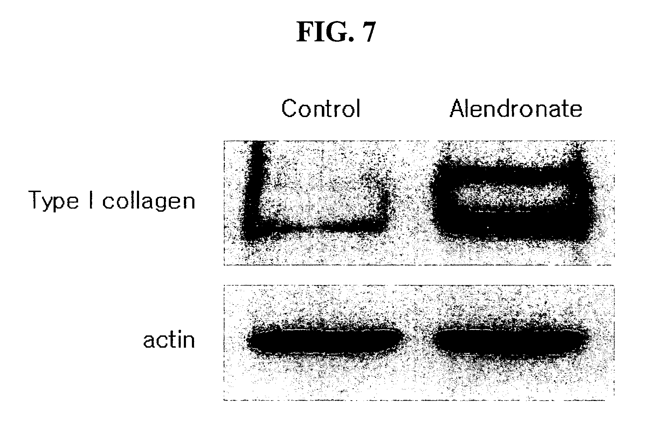

Moreover, in order to confirm whether the addition of other bisphosphonates than pamidronate provides the same effect of increasing collagen synthesis, fibroblasts were cultured in a medium containing alendronate. The results showed that, even in the case of addition of alendronate, the mRNA level of type 1 collagen and the production of type 1 collagen were remarkably increased in the same manner as in the case of addition of pamidronate (see FIGS. 6 and 7).

This suggests that the bisphosphonates of the present invention remarkably increase the synthesis of collagen (particularly type 1 collagen) in fibroblasts.

In another embodiment of the present invention, it was found that the bisphosphonates of the present invention have the effect of reducing wrinkles (one of skin aging phenomena) by increasing collagen synthesis in skin fibroblasts (see Table 2).

Accordingly, the inventive composition for increasing collagen synthesis comprising bisphosphonate as an active ingredient may be advantageously used in healing wounds requiring collagen synthesis, and in restoring a reduction in skin elasticity and the formation of wrinkles, which are associated with collagen deficiency, and also in treating gum decay, muscle pain, or damage of blood vessel walls.

The inventive composition for increasing collagen synthesis comprising bisphosphonate continuously increases collagen production by fibroblasts present in dermis, tissue and organs, etc. Thus, if animals including human beings are administered orally with the inventive composition, collagen production will be stably maintained, so that a reduction in the collagen synthesis of the skin damaged by aging or factors, such as ultraviolet rays, will be restored, thus giving the skin a tight and moist look, removing fine wrinkles and wrinkles, and restoring the skin elasticity.

Moreover, if the inventive composition is administered transdermally, it will rapidly reach fibroblasts present in dermis and continuously increase collagen production in the fibroblasts, so that a reduction in the collagen synthesis of the skin damaged by aging or factors, such as ultraviolet rays, will be restored, thus giving the skin a tight and moist look, removing fine wrinkles and wrinkles, and restoring the skin elasticity.

Thus, the inventive composition for increasing collagen synthesis comprising bisphosphonate may contain a suitable carrier, such that it is in the form of a pharmaceutical composition, particularly a dermatological composition or a cosmetic composition.

The inventive pharmaceutical composition for increasing collagen synthesis comprises the compound of formula (1), a pharmaceutically acceptable salt thereof or a hydrate thereof as an active ingredient, and a pharmaceutically acceptable carrier and optionally, other therapeutic components.

The inventive composition for increasing collagen synthesis comprising bisphosphonate as an active ingredient may be administered orally or parenterally and used in the form of a general medical formulation.

If the inventive composition is formulated in the form of a general medical product, it may be formulated using diluents or excipients, such as fillers, extenders, binders, wetting agents, disintegrants and surfactants, generally known in the art. Solid formulations for oral administration include tablets, pills, powders, granules and capsules, and such solid formulations are prepared by mixing bisphosphonate with at least one excipient, such as starch, calcium carbonate, sucrose, lactose or gelatin. In addition to the excipient, lubricants, such as magnesium stearate and talc, are used. Liquid formulations for oral administration include suspension, internal liquid, emulsion and syrup, and may be prepared using various excipients (e.g., wetting agents, sweetening agents, fragrant, preserving agents) in addition to commonly used simple diluents such as water and liquid paraffin. Formulations for parenteral administration include sterilized solution, non-aqueous solvent, suspending agent, emulsifier, freeze-drying agent, ointment or cream. Examples of the non-aqueous solvent or suspension solvent include propylene glycol, polyethylene glycol, vegetable oil such as olive oil, and injectable ester such as ethyloleate.

Also, the inventive composition may be administered parenterally, and the parentemal administration is performed by subcutaneous, intravenous or intramuscular injection. For the preparation of a formulation for parentemal administration, bisphosponate is mixed with a stabilizer or buffer in water so as to form solution or suspension, which is then formulated into a unit dosage form of an ampoule or vial.

As used herein, the term "active amount" refers to the amount of a compound which shows the effect of increasing collagen synthesis when administered to a patient. The daily dosage of bisphosphonate is preferably in a range of 0.05-20mg/kg, and more preferably 0.1-10 mg/kg.

The compound may be administered one or several times in the preferred daily dosage range. However, the dosage of bisphosphonate comprised in the inventive composition can be suitably chosen depending on an administration route, and the age, sex, body weight, individual property and disease condition of a subject to be administered with the compound. The inventive composition is not limited to particular formulations, administration routes and administration methods as far as it retains the inventive effects. In another aspect, the present invention provides an agent for treating skin wounds, which comprises bisphosphonate. The wound-healing agents comprising bisphosphonate, may be formulated into a medical product for skin application, particularly ointment or cream, and bisphosphonate may be added with the amount of 0.001- 10.0 wt%, and preferably 0.001-5.0 wt%, relative to the total weight of the formulation. If bisphosphonate is added at the amount of less than 0.001 wt%, an obvious effect may not be obtained, and if it is added at the amount of more than 10 wt%, it may not provide a further increase in its effect according to an increase in its content and may adversely affect the physical properties of the resulting product.

In still another aspect, the present invention provides cosmetics for the prevention and improvement of skin aging, which comprise the bisphosphonate.

The inventive cosmetics comprising the bisphosphonate are prepared by a conventional method known in the cosmetic field. The bisphosphonate may be added with the amount of 0.001-10.0 wt%, and preferably 0.001-5.0 wt%, relative to the total weight of the cosmetic formulation.

The inventive cosmetics for the prevention or improvement of skin aging may be used in the skin or hair, and prepared by mixing the inventive composition with a fundamental cosmetic composition (toner, cream, essence, cleansing foam, cleansing water, pack, or body oil), a makeup cosmetic composition (foundation, lipstick, mascara, or makeup base) or a hair cosmetic composition (shampoo, rinse, hair conditioner or hair gel), as well as with pharmaceutically or dermatologically acceptable excipients. The excipients include a pharmaceutically acceptable skin softener, skin penetration enhancer, colorant, flavoring agent, emulsifier, thickening agent, and solvent.

The inventive cosmetics for preventing or improving of skin aging, which comprise the bisphosphonate, may be applied to body sites requiring a reduction in wrinkles and an increase in elasticity, and preferably face, neck, hands, foot, arm, leg, and the like. The inventive cosmetics may be used daily for an undetermined period. Preferably, the use frequency and period of the cosmetics may be adjusted depending on the age and skin condition of a user and the concentration of the bisphosphonate.

In another further aspect, the present invention provides a method for increasing collagen synthesis *in vitro* using the bisphosphonate.

More concretely, the inventive method comprises the steps of: adding the bisphosphonate in certain cells or cell lines known to synthesize collagen, preferably fibroblasts or a culture thereof and culturing thereof.

The inventive method for synthesizing collagen will now be described as follows.

### a) First culture step of fibroblasts

Fibroblasts are first cultured on a culture dish. For this purpose, human epithelial cells which have been isolated by a conventional method are cultured in a medium while fibroblasts are selected from the medium. The selected fibroblasts are sub-cultured to obtain a culture.

### b) Second culture step of fibroblasts

The culture from the step a) is either two-dimensionally cultured in a culture dish (two-dimensional culture), or inoculated to a support capable of maintaining the three-dimensional form of the synthesized collagen and then three-dimensionally cultured (three-dimensional culture).

### c) Step of inducing collagen synthesis by addition of bisphosphonate

The fibroblasts are continued to culture in a medium containing bisphosphonate, thus inducing collagen synthesis by the fibroblasts.

At this step, a medium containing a substance known to induce collagen formation, such as vitamin C, in addition to the bisphosphonate, may be also used.

### d) Extraction step of synthesized collagen

The cultured support was collected, and collagen adhered on the support was isolated and extracted. At this time, the isolation of collagen adhered on the support is preferably performed by treatment with acids, but the isolation method is not limited to any particular methods. Furthermore, the isolated collagen may be used in a liquid or solidified state. However, it is preferably used in a solidified state in view of a convenience in storage and distribution. The solidification of collagen is preferably performed by a freeze-drying method or a precipitation method, and more preferably a method where the isolated collagen is precipitated by treatment with a compound containing sodium ions and then dried. However, the solidification method is not limited to any particular methods.

The culture of the fibroblasts is preferably performed under conditions of 1-10% (v/v) CO₂ and 30-40 °C, but not limited thereto.

Particularly, the porous support used in the three-dimensional culture is preferably a glycolic acid derivative with an average pore size of 300-500 µm and a porosity of 90%, and more particularly poly D,L-glycolic-co-lactic acid (PLGA).

Although the bisphosphonate is preferably added at a concentration of 1 nM-10 µM relative to the total volume of the fibroblast culture, but the addition amount of bisphosphonate is not limited to any amount.

According to the inventive synthesis method, collagen synthesis in fibroblasts is remarkably increased by the addition of bisphosphonate. Thus, the inventive method can be widely used in the effective preparation of products containing collagen.

Particularly, the collagen produced by the three-dimensional culture of fibroblasts has a three-dimensional structure. Thus, when it is administered to a living body, a possibility for an immune rejection response to occur is remarkably reduced.

In one embodiment of the present invention, fibroblasts were two-dimensionally or three-dimensionally cultured, and the effect of pamidronate on the mRNA and expression levels of type 1 collagen synthesized in the fibroblasts was determined. The results showed that pamidronate remarkably increased collagen synthesis not only in the two-dimensional culture of fibroblasts (see FIGS. 2 and 5) but also in the three-dimensional culture (see FIG. 3).

Thus, it was confirmed that the use of the bisphosphonates in collagen biosynthesis by the in vitro culture of fibroblasts resulted in an increase in the production yield of the collagen.

Furthermore, the present invention provides the use of bisphosphonate. More specifically, the present invention provides the use of bisphosphonate for the preparation of a composition or drug for increasing collagen synthesis, cosmetics for preventing or improving of skin aging or a wound-healing agent. The drug may contain a pharmaceutically acceptable carrier in addition to the bisphosphonate. Examples of the pharmaceutically acceptable carrier are as described above.

### BRIEF DESCRIPTION OF THE DRAWINGS

FIG. 1 is an electrophoresis photograph showing the effects of pamidronate and vitamin C on the mRNA level of type 1 collagen in the two-dimensional culture of fibroblasts isolated from human oral epithelium.
   1WC: a 1-week cultured control group;
   2WC: a 2-week cultured control group;
   3WC: a 3-week cultured control group;
   1WR: a 1-week cultured test group;
   2WR: a 2-week cultured test group; and
   3WR: a 3-week cultured test group.
FIG. 2 is a photograph showing the effects of pamidronate and vitamin C on the expression of type 1 collagen synthesized in the two-dimensional culture of fibroblasts isolated from human oral epithelium.
FIG. 3 is a photograph showing the effects of pamidronate and vitamin C on the expression of type 1 collagen synthesized in the three-dimensional culture of fibroblasts isolated from human dermis.
FIG. 4 is an electrophoresis photograph showing the effect of pamidronate on the mRNA level of type 1 collagen synthesized in the two-dimensional culture of fibroblasts isolated from human dermis.
FIG. 5 is a photograph showing the effect of pamidronate on the expression of type 1 collagen in the two-dimensional culture of fibroblasts isolated from human dermis.
FIG. 6 is an electrophoresis photograph showing the effect of alendronate on the mRNA level of type 1 collagen synthesized in the two-dimensional culture of fibroblasts isolated from human dermis.
FIG. 7 is a photograph showing the effect of alendronate on the expression of type 1 collagen in the two-dimensional culture of fibroblasts isolated from human dermis.

### BEST MODE FOR CARRYING OUT THE INVENTION

The present invention will hereinafter be described in further detail by examples. It will however be obvious to a person skilled in the art that the present invention is not limited to or by the examples.

### Example 1: Collagen synthesis by two-dimensional culture of oral fibroblasts using pamidronate

### 1-1) Isolation of oral fibroblasts

The epithelium naturally exuded from a patient who had been undergoing a surgical operation due to oral diseases was mixed with high-glucose DMEM (LG-DMEM, glucose 1g/L, GIBCO, MO, USA) medium, a first culture medium, and the mixture was pre-centrifuged to isolate cells. The cells were ultra-centrifuged using Percoll solution (SIGMA, MD, USA), thus isolating only nucleated cells. The nucleated cells isolated as described above cultured in an incubator under conditions of 5% (v/v) CO₂ and 37.5 °C while replacing a second culture medium (high-glucose DMEM medium containing 10% bovine fetal serum) at two-day intervals, and only fibroblasts were isolated from the culture medium.

### 1-2) Culture of oral fibroblasts

The isolated fibroblasts were cultured in said second culture medium under the same conditions for 2 days to saturate them. Then, the saturated fibroblasts were cultured in a second culture medium containing 1mM vitamin C and 100 ng/ml pamidronate (Hanlim Pharm. Co., Ltd., Korea) for each period of 1 week, 2 weeks and 3 weeks, while replacing the medium at 3-day intervals. As a control group, a second culture medium containing no vitamin C and pamidronate was used.

After the culture, cultures of the control group and the test group were collected from the respective culture dishes.

### 1-3) Analysis of collagen mRNA level

The collagen mRNA levels of the 1-week cultured control group (1WC), the 2-week cultured control group (2WC), the 3-week cultured control group (3WC), the 1-week cultured test group (1WR), the 2-week cultured test group (2WR) and the 3-week cultured group (3WR), which had been collected in Example 1-2), were measured and compared with each other.

Namely, total mRNA to be used in cDNA synthesis was isolated from the cells of the control and test groups using an RNeasy mini kit (QIAGEN, CA, USA). Then, the amount of the isolated total mRNA was calculated by measuring the absorbance at 260nm/280nm using an UV spectrophotometer (Eppendorf, Hamburg, Germany), thus quantifying the extracted mRNA and determining its purity (data not shown). Thereafter, 0.5 µg of the total mRNA was reacted with reverse transcriptase (Promega, WI, USA) using an oligo d(T) primer for one hour at 42 °C and for 5 minutes at 95 °C, and then the reaction was stopped at 4°C, thus synthesizing cDNA. The synthesized cDNA template was subjected to polymerase chain reaction (hereinafter, referred to as "PCR") using complementary primers of 5'-GACGAGACCAAGAACTG-3' (SEQ ID NO: 1) (sense) and 5'-CCATCCAAACCACTGAAACC-3' (SEQ ID NO: 2) (anti-sense). In the RT-PCR reaction, water was added to a reaction mixture of 0.5 µl of the template, 0.7 µl of each primer (10 pmole/µl) and PCR premix (Bioneer, Korea) to a final volume of 20 µl, and then, the aqueous mixture was pre-denatured at 94°C for 5 minutes using a thermal cycler and then subjected to 25 PCR cycles (each cycle consisting of 30 seconds at 94 °C, 30 seconds at 58 °C, and 30 seconds at 72 °C). Also, in the RT-PCR reaction, glyceraldehyde-3-phosphate dehydrogenase (GAPDH) was used as a positive control. The PCR product obtained from each sample was electrophoresed on agarose gel, and the electrophoresis results are shown in FIG. 1 for the comparison of collagen mRNA level between the samples. As shown in FIG. 1, in the control groups (1WC, 2WC and 3 WC), the mRNA level of type 1 collagen was gradually increased with an increase in culture time.

On the other hand, in the case of the test groups (1WR, 2WR and 3WR), the mRNA level of type 1 collagen observed in the 1-week cultured sample (1WR) was higher than that of the 3-week cultured sample (3WC), and all the test groups (1WR, 2WR and 3WR) showed a higher mRNA level than that of the 3-week cultured control group (3WC). This suggests that the addition of pamidronate provides an increase in the synthesis of mRNA of type 1 collagen.

### 1-4) Analysis of level of type 1 collagen protein

The levels of type 1 collagen in the 3-week cultured control group (3WC) and the 3-week cultured test group (3WR), which had been collected in Example 1-2), were compared with each other. For this purpose, each sample was subjected to Western blot analysis. In the Western blot analysis, each sample was electrophoresed on 8% SDS-PAGE gel, and the resulting collagen band was transferred to a nitrocellulose membrane and then treated with type 1 collagen monoclonal antibody (Abcam, Cambridge, UK) (see FIG. 2). In the Western blot analysis, actin was also used as a positive control group. As shown in FIG. 2, it could be found that, in the control group (3WC), the collagen protein level was very low, but in the test group (3 WR), the type 1 collagen protein was synthesized at a remarkably high level. This suggests that the addition of pamidronate provides a remarkable increase in collagen synthesis.

### Example 2: Collagen synthesis by three-dimensional culture of fibroblasts using pamidronate

### 2-1) Culture of dermal fibroblasts

A given amount of dermal tissue was excised from the skin of three normal persons. The excised human dermis from which an epidermal and fatty layer had been removed was finely cut and treated with collagenase so as to isolate dermis from epidermis. The isolated dermal tissue was cultured in 0.25% trypsin solution at 36 °C for 10 minutes. Then, the culture broth was vortexed to isolate fibroblasts. The isolated cells were collected and washed, and then cultured in a DMEM medium (GIBCO, MO, U.S.A.) containing 10% fetal bovine serum (FBS). At about 70% confluence, they were subcultured.

### 2-2) Three-dimensional culture of dermal fibroblasts

A PLGA-based, disc-shaped polymer support (Innotech., Korea) with a diameter of 10 mm, a thickness of 3 mm, an average pore size of 300-500 µm and a porosity of 90%, was used in three-dimensional culture. The support was treated with 100 %(v/v), 90 %(v/v), 70 %(v/v) and then 10 %(v/v) ethanol in order, at 4 °C for each treatment, and washed with PBS (phosphate buffered saline) and a second culture medium followed by drying. Next, 100 µl of a suspension of 1 x 10⁶ dermal fibroblasts cultured as described above was plated on the support and cultured in a culture dish with a diameter of 60 mm. After two hours, 6 ml of the second culture medium as described above was added to the cells, and the mixture was cultured in a shaking incubator (30rpm). Two days after the cell plating, the cells were supplied with a second culture medium containing 1 mM vitamin C and 100 nM pamidronate, at 3-day intervals, and cultured for 4 weeks. At this time, fibroblasts cultured in a second culture medium containing only vitamin C without pamidronate were used as a control group. After 4 weeks of the culture, the cultured cell-support composite was fixed in 10% neutral formalin for at least 8 hours, embedded in paraffin, cut into 5-µm thick sections, stained with haematoxylin-eosin and then observed under a microscope. The observation results are shown in FIG. 3.

As shown in FIG. 3, it could be found that in the case of addition of both vitamin C and pamidronate, the layer of collagen adhered to the surface of the PLGA support was much thicker than the case of addition of vitamin C alone (control group).

This suggests that pamidronate provides a remarkable increase in the collagen synthesis of fibroblasts even in three-dimensional culture.

### Example 3: Collagen synthesis by two-dimensional culture of dermal fibroblasts using pamidronate

In order to examine whether the single addition of pamidronate also has the effect of increasing the collagen synthesis of fibroblasts, the following test was performed:

Fibroblasts were isolated from skin dermis and subcultured, in the same manner as in Example 2. Then, the isolated fibroblasts were cultured in the second culture medium as described above under the same conditions for 2 days. Then, the cultured fibroblasts were supplied with a second culture medium containing 100 nM pamidronate at 3-day intervals and cultured in the medium for each period of 1 week, 2 weeks and 3 weeks. Also, fibroblasts cultured in a second culture medium containing 1mM vitamin C were used as a positive control group, and fibroblasts cultured in a secondary culture medium containing no vitamin C and pamidronate was used as a control group.

RT-PCR on each of the 1-week, 2-week and 3-week cultured control groups, the 1-week, 2-week and 3-week cultured test group administered with pamidronate, and the 1-week, 2-week and 3-week cultured positive control group administered with vitamin. C, was performed in the same manner as in Example 1-3). The mRNA levels of type 1 collagen in the samples, as measured in the RT-PCR reactions, were shown in FIG. 4 for comparison with each other. In addition, the expression level of type 1 collagen in each of the 3-week cultured control group, the 3-week cultured test group administered with pamidronate, and the 3-week cultured positive control group administered with vitamin C, were measured in the same manner as in Example 1-4), and the results are shown in FIG. 5 for comparison with each other.

As shown in FIG. 4, even when pamidronate was added alone, the synthesis of type 1 collagen mRNA was promoted as in the case of addition of vitamin C. Furthermore, in the case of addition of vitamin C, the mRNA level of type 1 collagen was increased rapidly in the initial stage of culture, but it was reduced with an increase in culture time. On the other hand, in the case of addition of pamidronate, the mRNA level of type 1 collagen was high regardless of culture period. This suggests that vitamin C and pamidronate all promote the expression of type 1 collagen mRNA, but the expression patterns are different between vitamin C and pamidronate. In addition, as shown in FIG. 5, it could be found that the single addition of pamidronate resulted in a remarkable increase in the expression level of type 1 collagen after three weeks of culture, as compared with the single addition of vitamin C. This suggests that, even in the single addition of pamidronate without vitamin C, the synthesis of type 1 collagen protein is remarkably increased.

### Example 4: Effect of alendronate on increase in synthesis of type 1 collagen

In order to examine whether other bisphosphonates than pamidronate promote the type 1 collagen synthesis of fibroblasts, the following test was performed using alendronate (Hanlim Pharm., Co., Ltd., Korea) in place of pamidronate. In other words, the same procedure as described in Example 3 was performed except that fibroblasts were cultured for one week using the same concentration of alendronate as a substitute for pamidronate. Also, fibroblasts cultured in a second medium containing no alendronate were used as a control group.

An electrophoresis photograph showing the mRNA level of type 1 collagen in the fibroblasts cultured in the culture medium containing alendronate is shown in FIG. 6, and a photograph showing the results of Western blot analysis for the type 1 collagen protein is shown in FIG. 7.

As shown in FIGS. 6 and 7, it could be found that even when the culture medium contained alendronate, the synthesis of type 1 collagen was remarkably increased as in the case of addition of pamidronate.

This suggests that other bisphosphonates than pamidronate also promote the synthesis of type 1 collagen.

### Preparation Examples 1 and 2 and Comparative Example: Cream comprising bisphosphonate compound

Each of creams was prepared by mixing pamidronate or alendronate with components given in Table 1 at specified mixing ratio.

**Table 1**

| Components | Preparation Example (wt%) | | Comparative Example |
|---|---|---|---|
| | 1 | 2 | |
| Pamidronate | 5 | - | - |
| Alendronate | - | 5 | - |
| Stearic acid | 15.0 | 15.0 | 15.0 |
| Cetanol | 1.0 | 1.0 | 1.0 |
| Glycerin | 5.0 | 5.0 | 5.0 |
| Propylene glycol | 3.0 | 3.0 | 3.0 |
| Tocopheryl acetate | 0.2 | 0.2 | 0.2 |
| Preservative | Qs | qs | qs |
| Perfume | Qs | qs | qs |
| Purified water | Balance | Balance | Balance |
| Total | 100 | 100 | 100 |

### Example 5: Wrinkle improving effect

The creams prepared in Preparation Examples 1 and 2 and Comparative Example 1 were measured for the effect of reducing wrinkles. Thirty adult women of ages 30 to 60 were selected and each of the creams was uniformly applied around the eyes of the selected women two times every day for two months, and then a reduction in crow's feet of the eyes was measured. The reduction in wrinkles after two months was rated visually on a scale of four grades (no reduction; slight reduction; moderate reduction; and significant reduction), and the results are given in Table 2 below.

**Table 2**

| | Reduction in cow's feet wrinkles (persons) | | | |
|---|---|---|---|---|
| | No reduction | Slight reduction | Moderate reduction | Significant reduction |
| Preparation Example 1 | 0 | 5 | 15 | 10 |
| Preparation Example 2 | 0 | 4 | 14 | 12 |
| Comparative Example | 25 | 3 | 1 | 1 |

As evident from Table 2, it could be found that the inventive creams containing bisphosphonate showed an excellent effect on a reduction in wrinkles, as compared with the nutritive cream according to Comparative Example.

### INDUSTRIAL APPLICABILITY

As described in the above, the inventive bisphosphonate provide an increase in the synthesis of type I collagen in fibroblasts. The inventive composition comprising the bisphosphonate promotes collagen synthesis, and thus, when it is applied to the skin, it reduces wrinkles of the skin and makes the skin elastic. For this reason, the inventive composition will be useful as cosmetics for the prevention or improvement of skin aging or a wound-healing agent. Furthermore, the inventive composition can remarkably increase the collagen synthesis of fibroblasts *in vitro,* and thus, can be effectively used in the production of products containing collagen.

## Claims

1. The use of bisphosphonate for increasing collagen synthesis, the bisphosphonate being selected from the group consisting of compounds of the following formula 1, pharmaceutically acceptable salts thereof and hydrates thereof: 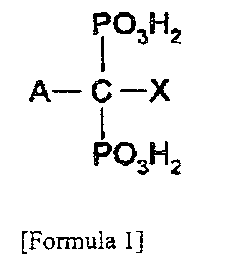 wherein A is hydrogen, hydroxy or halogen; and X represents one of the following: (a) an alkyl with 1-6 carbon atoms, which may be substituted with amino, alkylamino, dialkylamino or heterocyclyl; (b) halogen; (c) arylthio, and preferably an arylthio substituted with halogen; (d) a cycloalkylamino with 5-7 carbon atoms; and (e) a saturated 5- or 6-membered nitrogen-containing heterocyclyl with 1-2 heteroatoms.

2. The use of bisphosphonate of Claim 1 for the preparation of a wound-healing agent.

3. The use of bisphosphonate of Claim 1 for the preparation of cosmetics for the prevention or improvement of skin aging.

4. The use of any one of Claims 1 to 3, wherein the bisphosphonate is one selected from the group consisting of 4-amino-1-hydroxybutylidene-1,1-bisphospnonic acid (alendronic acid); N-methyl-4-amino-1-hydroxybutylidene-1,1-bisphosphonic acid; 4-(N,N-dimethyla.mino)-1-hydroxybutylidene-1,1-bisphosphonic acid; 3-amino-1-hydroxypropylidene-1,1-bisphysphonic acid (pamidronic acid); 3-(N-methyl-N-pentyl)-amino-1-hydroxypropane-1,1-bisphosphonic acid (ivandronic acid); 1-hydroxy-3-(N-methyl-N-pentylamino)-propylidene-1,1-bisphosphonic acid; 1-hydroxy-2-[3-pyridinyl]-ethylidene-1,1-bisphosphonic acid (licedronic acid); 4-(hydroxymethylene-1,1-bisphosphonic acid)-piperidine; cycloheptylaminomethylene-1,1-bisphosphonic acid (cimadronic acid); 1,1-dichloromethylene-1,1-bisphosphonie acid (clodronic acid); 1-hydroxy-3-(1-pyrolidinyl)-propylidene-1,1-bisphosphonic acid (EB- 105 3); 1-hydroxyethylidene-1,1-bisphosphonic acid (etidronic acid); 6-amino-1-hydroxyhexylidene-1,1-bisphosphonic acid (neridronic acid); 3-(dimethylamino)-1-hydroxypropylidene-1,1-bisphosphonic acid (olpadronic acid); [2-(2-pyridinyl)-ethylidene]-1,1-bisphosphonic acid (piridronic acid); 1-(4-chlorophenylthio)methylidene-1,1-bisphosphonic acid (tiludronic acid); 1-hydroxy-2-(1H-imidazol-1-yl)-ethylidene-1,1-bisphosphonic acid (zoledronic acid); (1-hydroxy-2-imidazo-(1,2-a)pyridin-3-ylethylidene]-bisphosphonic acid; pharmaceutically acceptable salts thereof and hydrates thereof.

5. The use of any one of Claims 1 to 3, wherein the bisphosphonate is one selected from the group consisting of ivandronic acid, clodronic acid, etidronic acid, licedronic acid, zoledronic acid, tiludronic acid, pamidronic acid, cimadronic acid, alendronic acid, pharmaceutically acceptable salts thereof and hydrates thereof.

6. The use of any one of Claims 1 to 3, wherein the bisphosphonate is one selected from the group consisting of pamidronic acid, alendronic acid, pharmaceutically acceptable salts thereof and hydrates thereof.

7. A method for increasing collagen synthesis using bisphosphonate, the method comprises the steps of:
(a) culturing fibroblasts; and
(b) adding bisphosphonate to the fibroblast culture from step (a) so as to induce collagen synthesis.

8. The method of Claim 7, wherein the bisphosphonate is one selected from the group consisting of 4-amino-1-hydroxybutylidene-1,1-bisphospnonic acid (alendronic acid); N-methyl-4-amino-1-hydroxybutylidene-1,1-bisphosphonic acid; 4-(N,N-dimethylamino)-1-hydroxybutylidene-1,1 -bisphosphonic acid; 3-amino-1-hydroxypropylidene-1,1-bisphosphonic acid (pamidronic acid); 3-(N-methyl-N-pentyl)-amino-1-hydroxypropane-1,1-bisphosphonic acid (ivandronic acid); 1-hydroxy-3-(N-methyl-N-pentylamino)-propylidene-1,1-bisphosphonic acid; 1-hydroxy-2-[3-pyridinyl]-ethylidene-1,1-bisphosphonic acid (licedronic acid); 4-{hydroxymethylene-1,1-bisphosphonic acid)-piperidine; cycloheptylaminomethylene-1,1-bisphosphonic acid (cimadronic acid); 1,1-dichloromethylene-1,1-bisphosphonic acid (clodronic acid); 1-hydroxy-3-(1-pyrrolidinyl)-propylidene-1,1-bisphosphonic acid (EB-1053); 1-hydroxyethylidene-1,1-bisphosphonic acid (etidronic acid); 6-amino-1-hydroxyheXylidene-1,1-bisphosphonic acid (neridronic acid); 3-(dimethylamino)-1-hydroxypropylidene-1,1-bisphosphonic acid (olpadronic acid); [2-(2-pyridinyl)-ethylidene]-1,1-bisphosphonic acid (piridronic acid); 1-(4-chlorophenylthio)methylidene-1,1-bisphosphonic acid (tiludronic acid); 1-hydzoxy-2-(1H-imidazol-1-yl)-ethylidene-1,1-bisphosphonic acid (zoledronic acid); [1-hydroay-2-imidazo-(1,2-a)pyridin-3-ylethylidene]-bisphosphonic acid; pharmaceutically acceptable salts thereof and hydrates thereof.

9. The method of Claim 7, wherein the bisphosphonate is one selected from the group consisting of ivandronic acid, clodronic acid, etidronic acid, licedronic acid, zoledronic acid, tiludronic acid, pamidronic acid, cimadronic acid, alendronic acid, pharmaceutically acceptable salts thereof and hydrates thereof.

10. The method of Claims 7, wherein the bisphosphonate is one selected from the group consisting of pamidronic acid, alendronic acid, pharmaceutically acceptable salts thereof and hydrates thereof.

11. The method of Claim 7, which further comprises adding vitamin C to the fibroblast culture.

12. The method of any one of Claims 7 to 11, wherein the step a) is performed either by two-dimensional culture in a culture dish or by three-dimensional culture in a porous support.

13. The method of Claim 12, wherein the porous support is a glycolic acid derivative with an average pore size of 300-500 µm and a porosity of 90%.

14. The cosmetics for the prevention or improvement of skin aging, which comprises bisphosphonate of Claim 1.
